# EUROPEAN PATENT APPLICATION

(11) **EP 0 824 974 A1**
(43) Date of publication of application: **25.02.1998**
(21) Application number: 97902671.3
(22) Date of filing: 13.02.1997
(51) Int. Cl.: B08B 3/02, B08B 3/12, B08B 9/20, A61L 2/18

(54) **METHOD AND APPARATUS FOR STERILIZING CAPS**

(30) Priority: 20.02.1996 JP 31893/96
(71) Applicant: Mitsubishi Heavy Industries, Ltd., Tokyo 100 (JP)
(72) Inventor: YAMAGUCHI, Shogo, Nagoya Machinery Works,, Iwatsuka-cho Nakamura-ku Achichi-ken 453 (JP); TAKENAKA, Yoshiaki, Nagoya Machinery Works,, Iwatsuka-cho, Nakamura-ku, Aichi-ken 453 (JP); YAMAGUCHI, Yukio Churyo Engin. Kabushiki Kaisha,, Nagoya-shi Aichi-ken 453 (JP)
(74) Representative: Henkel, Feiler, Hänzel & Partner
(86) International application number: JP9700379
(87) International publication number: WO9730799

(57) **Abstract**

A method of completely sterilizing caps maintaining safety to human body, and an apparatus therefor. A predetermined number of caps (C) are changed into carriers (20) which are then successively passed through three chambers (9, 10, 11) in a sterilizer box (2) equipped with an exhaust fan (5). In these chambers are carried out pre-washing using recycled ozone water, main sterilization using high-ozone-concentration water and rinsing using low-ozone-concentration water in this order.

## Description

### BACKGROUND OF THE INVENTION:

### Field of the Invention:

The present invention relates to a method and apparatus for sterilizing caps for containers of PET (polyethylene terephthalate) bottles etc.

### Description of the Prior Art:

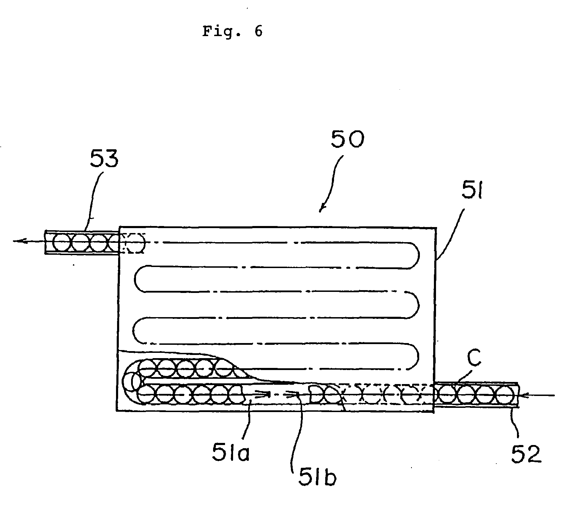

Fig. 6 is a plan view showing a schematic construction of a prior art sterilizing apparatus 50 for sterilizing caps for containers of PET bottles etc. to be filled with a drinking water etc. by use of a low concentration ozone water so as not to be harmful to the human body. In the figure, letter C designates caps, numeral 51 designates a sterilizing tank for sterilizing caps by ejecting the low concentration ozone water, numeral 52 designates a supply air conveyor for carrying the caps C, removed from containers by a cap separator (not shown) and aligned in a same posture in a row, to the sterilizing tank 51 and numeral 53 designates a discharge air conveyor for receiving the caps C, after sterilized, from the sterilizing tank 51 and conveying them to a cap fastening apparatus (not shown).

Within the sterilizing tank 51, there are provided a serpentine guide groove 51a for guiding the caps C so as to proceed from an inlet to an outlet, and downward and upward nozzles 51b which are disposed inclinedly toward a proceeding direction of the caps C above and below said guide groove 51a with a predetermined interval between each of the nozzles 51b. Also provided at the sterilizing tank 51 is a single hinged canopy opening upwardly, by which, if opened, the guide groove 51a can be opened.

The low concentration ozone water is adjusted to a predetermined ozone concentration by an ozone water producing apparatus (not shown) and is ejected from the nozzles 51b and circulated by a pump. At the discharge air conveyor 53, a water removing apparatus by use of a clean air (not shown) is provided.

The caps C are supplied into the sterilizing tank 51 pushedly by the supply air conveyor 52 to run within the sterilizing tank 51 guidedly by the guide groove 51a, and while so running, they are steeped for a predetermined time to be sterilized in the ozone water ejected from the nozzles 51b and then is discharged from the outlet onto the discharge air conveyor 53. It is to be noted that the caps C are assisted to run within the sterilizing tank 51 by force of the ozone water ejected by the nozzles 51b inclinedly toward the proceeding direction. The caps C, after coming out of the sterilizing tank 51, is removed of water by blowing of a clean air and then is fed to the cap fastening apparatus of a next step.

It is to be noted that, in case of a change of kind of cap accompanying with a change of kind of liquid to be filled in containers of PET bottles etc., the canopy of the sterilizing tank 51 is opened and all the caps C remaining in the sterilizing tank 51 are taken out manually so that the sterilizing tank 51 becomes once vacant and then next work is started with caps supplied for a new kind of liquid.

The mentioned prior art is a cap sterilizing apparatus using a low concentration ozone water (ozone content is approximately 1 ppm) so as not to be harmful to the human body and is not sufficient for a sterilization requiring a complete sterilization, so in order to effect a complete sterilization, it is necessary to make the ozone concentration higher (to ozone content of 10 to 20 ppm) with a result that there arises a problem of safety to the human body.

It is therefore an object of the present invention to provide a cap sterilizing method and apparatus which can achieve a complete sterilization and are safe to the human body.

### SUMMARY OF THE INVENTION:

With a view to solve the problem in the prior art, the present invention provides a cap sterilizing method having feature as follows: that is, the cap sterilizing method according to the present invention comprises steps of; receiving a predetermined number of supplied caps in a carrier; and causing said carrier to pass sequentially three chambers disposed in a sterilizer box having an exhaust fan, wherein a pre-washing using a recycled ozone water in a first chamber of said three chambers, a main sterilizing using a high concentration ozone water in a second chamber of same and a rinsing using a low concentration ozone water in a third chamber of same are carried out in said order.

Also, in order to solve the problem in the prior art, the present invention provides a following cap sterilizing apparatus; that is, the cap sterilizing apparatus according to the present invention is for sterilizing using ozone water in a sterilizer box having an exhaust fan and an ozone killer and comprises; a pre-washing chamber having a spray nozzle; a main sterilizing chamber having an ejecting nozzle and a ultrasonic oscillator; and a rinsing chamber having a spray nozzle and an air nozzle, all said chambers being disposed in said sterilizer box, and comprises further; an ozone water producing apparatus for supplying a low concentration ozone water to said spray nozzle of said rinsing chamber and a high concentration ozone water to said ejecting nozzle; a pump for feeding with pressure an ozone water stored in a lower portion of said rinsing chamber to said spray nozzle of said pre-washing chamber; a clean air source for supplying a clean air to said air nozzle; and a carrier receiving a predetermined number of supplied caps and moving in each said chamber in said order.

The cap sterilizing apparatus according to the present invention preferably comprises a following carrier moving means so that there is a least influence given by an elongation occurring at a double pitch chain; that is, the carrier moving means consists of; two pairs of coaxial drive sprockets, each pair being disposed near an inlet and an outlet, respectively, of said sterilizer box; two indexing drive units which rotate coaxially with each other and of which each output shaft is connected to a sprocket shaft each of said two pairs of drive sprockets; and a double pitch chain wound around said two pairs of drive sprockets and moving while supporting a plurality of said carriers.

### BRIEF DESCRIPTION OF THE DRAWINGS:

Fig. 1 is a perspective view of a cap sterilizing apparatus of a first embodiment according to the present invention.

Fig. 2 is a cross sectional view seen from a front of said apparatus.

Fig. 3 is a perspective view of a carrier and a pusher used in said apparatus.

Fig. 4 is a perspective view of a cap to be sterilized by said apparatus.

Fig. 5 is a perspective view of a carrier moving means of a cap sterilizing apparatus of a second embodiment according to the present invention.

Fig. 6 is a plan view of a prior art cap sterilizing apparatus.

### DESCRIPTION OF THE PREFERRED EMBODIMENTS:

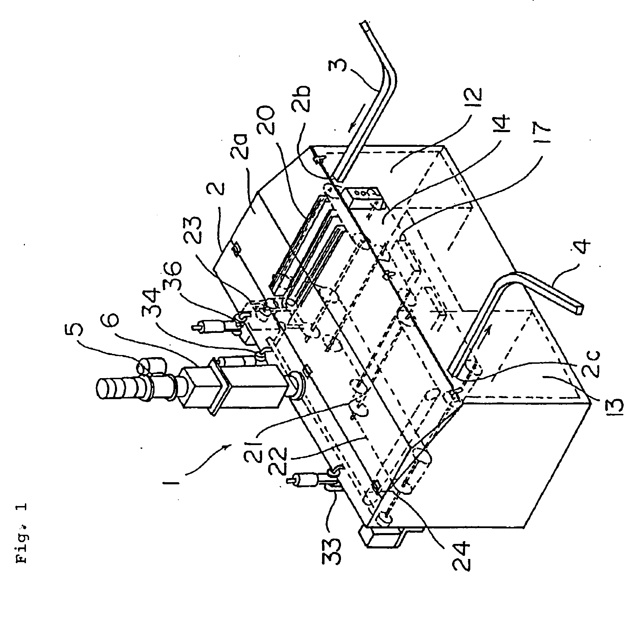

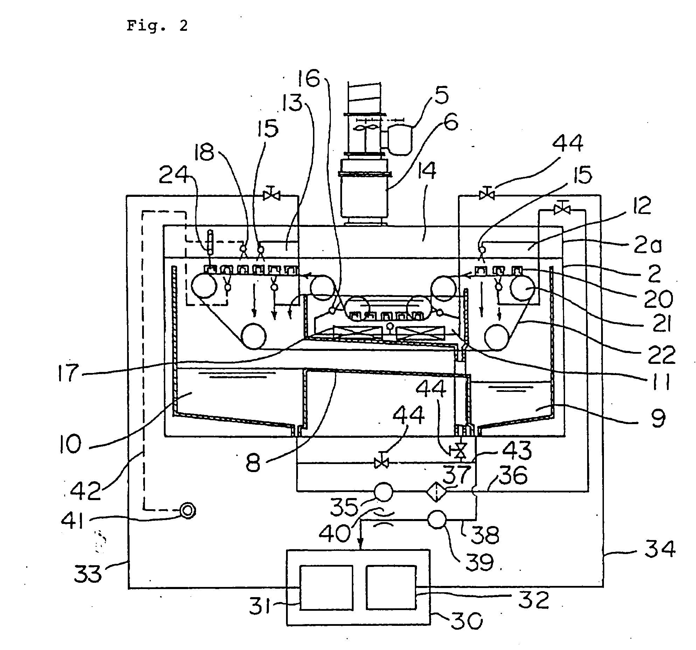

In Figs. 1 and 2 which show a cap sterilizing apparatus 1 of a first embodiment according to the present invention, numeral 2 designates a sterilizer box which is of a gas tight structure and is disposed in an aseptic room, numeral 2a designates a canopy of the sterilizer box 2 which opens upwardly, numeral 3 (Fig. 1) designates a supply air conveyor for carrying caps C, aligned in a single row by a cap separator (not shown), to an inlet 2b of the sterilizer box 2, numeral 4 (Fig. 1) designates a discharge air conveyor for carrying the caps C to a cap fastening apparatus (not shown) from an outlet 2c of the sterilizer box 2, numeral 5 designates an exhaust fan connecting to an upper portion of the sterilizer box 2, and numeral 6 designates an ozone killer disposed between the exhaust fan 5 and the sterilizer box 2.

Numerals 9, 10 and 11 (Fig. 2) designate ozone water tanks disposed fixedly within the sterilizer box 2, wherein a dirty ozone water tank 9 of an inlet side and a recycled ozone water tank 10 of an outlet side are divided by a weir 8 of a wide width and an ozone water tank 11 of a central upper portion is a high concentration ozone water tank. A pre-washing chamber 12 is formed above the dirty ozone water tank 9 of the inlet side and a rinsing chamber 13 is formed above the recycled ozone water tank of the outlet side, and a main sterilizing chamber 14 is formed, mainly by the ozone water tank 11, fixedly in a suspended state between the pre-washing chamber 12 and the rinsing chamber 13.

At an upper portion of the pre-washing chamber 12 and the rinsing chamber 13, respectively, a plurality of upward and downward spray nozzles 15 are provided, and within the high concentration ozone tank 11 of the main sterilizing chamber 14, a plurality of upward and lateral ejecting nozzles 16 and a ultrasonic oscillator 17 are provided. Also, at an upper portion of an outlet side of the rinsing chamber 13, there is formed a water removing space where a plurality of upward and downward air nozzles 18 are provided.

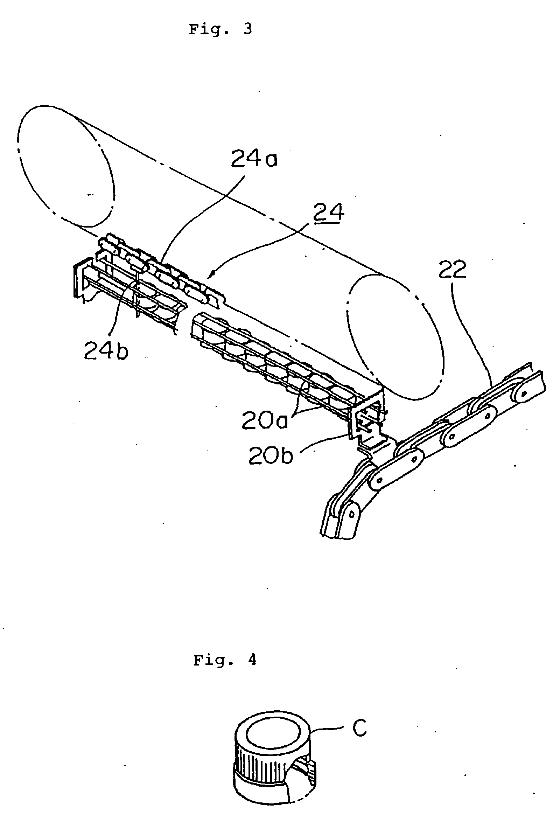

Numeral 20 (Fig. 3) designates a carrier which moves while holding a predetermined number of caps C. Said carrier 20 is formed by a plurality (six in Fig. 3) of round bars 20a, with each of their end portions fixed to a frame 20b, for restraining upper and lower faces and side faces each of the caps C and a terminal end each of the round bars 20a on a side into which the caps C are inserted is slightly bent outwardly so that the caps C are received easily into the carrier 20.

Numeral 21 (Fig. 1) designates plural pairs (eight pairs in Fig. 1) of sprockets, each pair having coaxially rotating two sprockets, numeral 22 designates two double pitch chains, each wound around the sprockets 21 to run along a side wall of the sterilizer box 2, and numeral 23 (Fig. 1) designates an index motor for driving sprockets 21 at two places of upper portions of the inlet 2b side and the outlet 2c side. Thus, the carrier 20 with its both ends being supported by the double pitch chains 22 can move intermittently in a rectangular direction relative to its longitudinal direction.

Numeral 24 designates a pusher disposed at a position of the outlet 2c, said pusher being an endless chain 24a (Fig. 3) and having a pin 24b fixed thereto by which the caps C within the carrier 20 moved to the position of the outlet 2c are pushed out onto the discharge air conveyor 4.

Numeral 30 (Fig. 2) designates an ozone water producing apparatus disposed outside of the sterilizer box 2, said ozone water producing device 30 consisting of a low concentration ozone water producing apparatus 31 and a high concentration ozone water producing apparatus 32, wherein a low concentration ozone water (ozone content is approximately 1 ppm) is fed with pressure to the spray nozzles 15 of the rinsing chamber 13 from the low concentration ozone water producing apparatus 31 via a conduit 33 and a high concentration ozone water (ozone content is 10 to 20 ppm) is fed with pressure to the ejecting nozzles 16 of the main sterilizing chamber 14 (the high concentration ozone water tank 11) from the high concentration ozone water producing apparatus 32 via a conduit 34. Also, an ozone water is fed with pressure to the spray nozzles 15 of the pre-washing chamber 12 from the recycled ozone water tank 10 of a lower portion of the rinsing chamber 13 via a pump 35, a filter 37 and a conduit 36. In Fig. 2, numeral 38 designates a return conduit for returning the ozone water to the ozone water producing apparatus 30 from the dirty ozone water tank 9 of a lower portion of the pre-washing chamber 12, numeral 39 designates a pump disposed in the return conduit 38, numeral 40 designates a cooler and numeral 41 designates a clean air supply source for supplying a clean air with pressure to the air nozzles 18 of the upper portion and the lower portion of the outlet side of the rinsing chamber 13 via a conduit 42. Also, numeral 43 designates a conduit for connecting the lower portion of the rinsing chamber 13 and the high concentration ozone water tank 11 to the return conduit 38 and numeral 44 designates an open/close valve provided at each of the conduits.

Next, function of the cap sterilizing apparatus as so constructed is described.

Caps C, removed from containers by a cap separator (not shown) and aligned in a single row with upper and lower sides of each cap being arrayed, are carried by the supply air conveyor 3 to be fed into a stationary carrier 20 from the inlet 2b of the sterilizer box 2. Upon a predetermined number (24 for example) of the caps C being so fed, the carrier 20 moves by one pitch of the carrier 20 in a rectangular direction relative to the row of the caps C and then stops.

At the pre-washing chamber 12, the caps C receive upward and downward spraying of a recycled ozone water from the spray nozzles 15 to become wet and be pre-washed so that dirt is removed therefrom. With a predetermined time (about 12 seconds for example) thereafter, the caps C proceed to the main sterilizing chamber 14 and go down to be steeped in the high concentration ozone water within the high concentration ozone water tank 11, and while receiving upward and lateral ejection of the high concentration ozone water from the ejecting nozzles 16 and receiving vibration caused at a lower portion by the ultrasonic oscillator 17, the caps C are exposed to a main sterilization for a predetermined time (about 36 seconds for example). Then, they go up to move to the rinsing chamber 13 and are rinsed for a predetermined time (about 12 seconds for example) by spraying of a low concentration ozone water from the spray nozzles 15, same as in the pre-washing chamber 12. Then, after receiving upward and downward blowing of a clean air from the air nozzles 18 and being removed of sticking water (for about 2 minutes in total for example), the caps C are discharged onto the discharge air conveyor 4 from the outlet 2c by the pin 24b of the pusher 24 which turns intermittently.

It is to be noted that the high concentration ozone water ejected at the main sterilizing chamber 14 overflows from the high concentration ozone water tank 11 toward the rinsing chamber 13 and is stored in the recycled ozone water tank 10. In this recycled ozone water tank 10, the high concentration ozone water mixes with the low concentration ozone water which has been used in the rinsing chamber 13 and is reduced of its concentration approximately by halt to become a recycled ozone water of ozone content of 5 to 10 ppm. This recycled ozone water is fed with pressure to the spray nozzles 15 of the pre-washing chamber 12 to be used for the pre-washing, as mentioned above, and a surplus ozone water flows over the weir 8 to be stored directly in the dirty ozone water tank 9 of the pre-washing chamber 12 side.

Air separating from the ozone water and including a noxious ozone gas is drawn by the exhaust fan 5 to be made innoxious by the ozone killer 6 and be discharged outside and, the sterilizer box 2 being thus maintained in a negative pressure, there occurs no leakage of the ozone gas outside of the sterilizer box 2.

In case of a change of kind of cap accompanying with a change of kind of liquid to be filled, supply of the caps C is stopped and all the caps C within the sterilizer box 2 are discharged out of the sterilizer box 2, thus there is no need of opening the canopy 2a of the sterilizer box 2.

It is to be noted that if supply of the caps C into the sterilizer box 2 is done onto the supply air conveyors provided in two or more rows as well as if change of carrying pitch of the carrier 20 and change of increasing the pusher and the discharge air conveyor, etc. are added, it is of course possible to increase the sterilizing capacity of the caps.

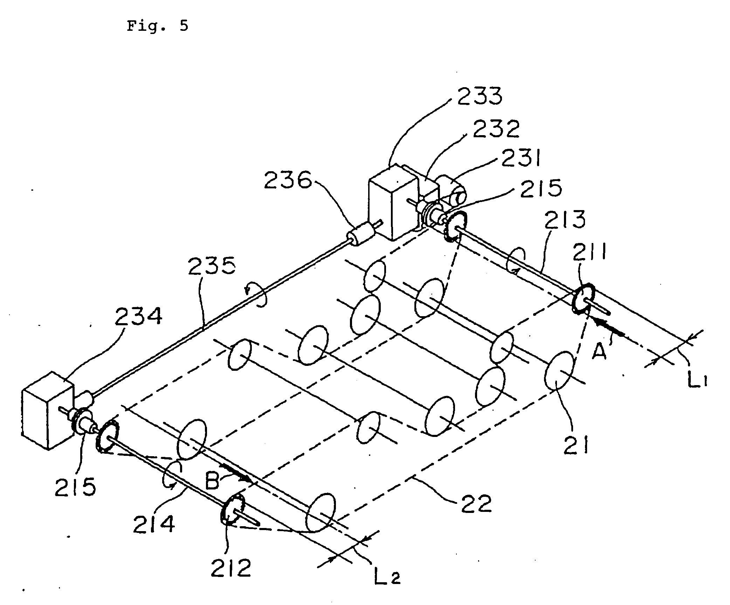

Fig. 5 shows a carrier moving means of a cap sterilizing apparatus of a second embodiment according to the present invention and in this embodiment, arrangement of sprockets 21 and double pitch chains 23 is same as in the first embodiment and only difference is a means for driving the sprockets, other portions being same as in the first embodiment.

In Fig. 5, numeral 231 designates a drive motor, numeral 232 designates a clutch with breaking unit, numeral 233 and 234, respectively, designates an indexing (drive) unit, numeral 235 designates a connecting shaft for connecting input shafts of the two indexing units 233 and 234, numerals 211 and 212 designate two pairs of drive sprockets, one pair being disposed on the inlet side and the other on the outlet side, out of the above-mentioned eight pairs of the sprockets 21, numeral 213 and 214, respectively, designates a sprocket shaft of the drive sprockets 211 and 212, and numeral 215 designates a flange coupling for connecting the sprocket shafts 213 and 214 and an output shaft of the indexing units 233 and 234, respectively.

Arrow A, B of Fig. 5 shows a longitudinal central position of the supply air conveyor 3 and the discharge air conveyor 4 (see Fig. 1), respectively, for supplying and discharging the caps C, and the horizontal length L₁, L₂ between the longitudinal central position of the conveyor and the sprocket shaft 213, 214, respectively, is made shorter as much as possible (about 100 mm for example.

The drive motor 231 drives an input shaft of the clutch with breaking unit 232 via a belt, an output shaft of the clutch with breaking unit 232 drives an input shaft of the indexing unit 233 of a double shaft type and an output shaft of said indexing unit 233 and an input shaft of another indexing unit 234 are connected by the connecting shaft 235 via a coupling 236 (a muff type coupling). Also, the flange coupling 215 has bolt holes of its one flange being bored in oblong holes so that angle adjustment in a rotational direction can be done within the range of these oblong holes.

The indexing unit 233, 234 is of an intermittently indexing type that repeats actions, each action to index a constant angle and then to stop for a while. A pitch time of indexing is adjusted to a predetermined pitch time (about 12 seconds for example) and a stopping time is about 3/4 of the pitch time which is long enough and delivery and receipt of the caps C at the supply air conveyor 3 and the discharge air conveyor 4 is done during said stopping time. The drive motor 231 is operated continuously in order to avoid an inching motion. The clutch with breaking unit 232 is used for intercepting transmission between the drive motor 231 and the indexing unit 233, 234 when there occurs a need of changing a movement time of the carrier 20 (see Fig. 1) in relation to devices disposed upstream through downstream of the present sterilizing apparatus.

The drive sprocket 211, 212 stops at a specific position of posture indexed by the indexing unit 233, 234 and thus each link of the double pitch chain 22 meshed with and wound around said drive sprocket 211, 212 and the carrier 20 supported at intervals on said link can also stop at a specific position. In order to cause the carrier 20 to stop at position of the inlet 2b and the outlet 2c so that a longitudinal center line of the carrier 20 coincides with that of the supply air conveyor 3 and the discharge air conveyor 4, respectively, the sprocket shaft 213, 214 is rotated in advance so as to be adjusted to a right place by the flange coupling 215. It is necessary to make a deviation between both of said center lines smaller as much as possible (± about 1 mm for example) in order to carry out a smooth delivery and receipt of the caps C.

Generally there occurs an elongation of chain while it is used. While the link of chain at a portion meshing with the drive sprocket is restrained of its position, the link at a portion apart from the drive sprocket is not restrained and has a freedom of moving its position with the range of length of elongation. As for the elongation of chain, even if an initial elongation removal treatment is applied in advance, it will be necessary to have an allowance of approximately 0.75%, thus the link of chain at a portion apart from the drive sprocket has a considerably large freedom of moving its position. According to the apparatus of the present invention, the drive sprocket 211, 212 disposed at two place near the supply air conveyor 3 and the discharge air conveyor 4 restrains the double pitch chain 22, thus even if an elongation occurs at the double pitch chain 22, there is only a least influence given by the position both of the conveyors 3 and 4 on the position of the link of the double pitch chain 22, which can be easily included in a desired limit of error.

According to the sterilizing apparatus of the present invention, following effect can be obtained:
(1) A multitude of caps, gathered and moved by the carrier, are steeped in the ejected high concentration ozone water for a sufficient time at the main sterilizing chamber and are exposed to vibration caused by the ultrasonic means, thereby a sterilization to a completely sterilized state can be effected.
(2) The sterilization is done within the closed sterilizer box, and gas separating from the ozone water and including ozone gas therein is absorbed to be removed of the ozone gas by the ozone killer and then is discharged outside, and also the caps are rinsed by the low concentration ozone water and removed of sticking water and then are discharged out of the sterilizer box, thereby there occurs no harm due to ozone and a safety is secured.
(3) There is no specific need of using a normal ozone water for the pre-washing but the recycled ozone water after used for the rinsing can be used, thereby saving of energy cost can be achieved.
(4) The drive sprocket of the double pitch chain is disposed at two places near the supply air conveyor and the discharge air conveyor, thereby even if a slight elongation occurs at the double pitch chain, there occurs only a least influence given on the carrier stop position and on a deviation of alignment both of the conveyors.

## Claims

1. A cap sterilizing method characterized in comprising steps of; receiving a predetermined number of supplied caps in a carrier (20); and causing said carrier (20) to pass sequentially three chambers (12, 13, 14) disposed in a sterilizer box (2) having an exhaust fan (5), wherein a pre-washing using a recycled ozone water in a first chamber (12) of said three chambers, a main sterilizing using a high concentration ozone water in a second chamber (13) of same and a rinsing using a low concentration ozone water in a third chamber (14) of same are carried out in said order.

2. A cap sterilizing apparatus for sterilizing using ozone water in a sterilizer box (2) having an exhaust fan (5) and an ozone killer (6), characterized in comprising; a pre-washing chamber (12) having a spray nozzle (15); a main sterilizing chamber (14) having an ejecting nozzle (16) and a ultrasonic oscillator (17); and a rinsing chamber (13) having a spray nozzle (15) and an air nozzle (18), all said chambers being disposed in said sterilizer box (2), and characterized in comprising further; an ozone water producing apparatus (30) for supplying a low concentration ozone water to said spray nozzle (15) of said rinsing chamber (13) and a high concentration ozone water to said ejecting nozzle (16); a pump (35) for feeding with pressure an ozone water stored in a lower portion of said rinsing chamber (13) to said spray nozzle (15) of said pre-washing chamber (12); a clean air source (41) for supplying a clean air to said air nozzle (18); and a carrier (20) receiving a predetermined number of supplied caps and moving in each said chamber (12, 13, 14) in said order.

3. A cap sterilizing apparatus as claimed in Claim 2, characterized in comprising a carrier moving means consisting of; two pairs of coaxial drive sprockets (211, 212), each pair being disposed near an inlet and an outlet, respectively, of said sterilizer box (2); two indexing drive units (233, 234) which rotate coaxially with each other and of which each output shaft is connected to a sprocket shaft (213, 214) each of said two pairs of drive sprockets (211, 212); and a double pitch chain (22) wound around said two pairs of drive sprockets (211, 212) and moving while supporting a plurality of said carriers (20).
